# EUROPEAN PATENT SPECIFICATION

(11) **EP 3 424 964 B1**
(45) Date of publication and mention of the grant of the patent: **02.08.2023**
(21) Application number: 17759472.8
(22) Date of filing: 24.01.2017
(51) Int. Cl.: C08F 216/18, A61L 33/06, A61L 31/10, C08F 216/14, A61L 29/10

(54) **COPOLYMER, ANTITHROMBOTIC COATING AGENT USING SAME AND MEDICAL DEVICE**
COPOLYMER, ANTITHROMBOTISCHES BESCHICHTUNGSMITTEL DAMIT UND MEDIZINISCHE VORRICHTUNG
COPOLYMÈRE, AGENT DE REVÊTEMENT ANTITHROMBOTIQUE L'UTILISANT ET DISPOSITIF MÉDICAL

(30) Priority: 29.02.2016 JP 2016037277
(43) Date of publication of application: 09.01.2019
(73) Proprietor: Maruzen Petrochemical Co., Ltd., Tokyo 104-8502 (JP); National University Corporation Yamagata University, Yamagata-shi, Yamagata 990-8560 (JP)
(72) Inventor: TANAKA Masaru, Yonezawa-shi Yamagata 992-8510 (JP); YOSHIDA Norihiro, Ichihara-shi Chiba 290-8503 (JP)
(74) Representative: Beckmann, Claus
(86) International application number: PCT/JP2017/002199
(87) International publication number: WO 2017/150000

(56) References cited:
- WO-A1-2006/118278
- WO-A1-2008/041549
- WO-A2-2014/163626
- JP-A- 2005 002 193
- JP-A- 2009 167 282
- JP-A- 2011 052 079
- JP-A- 2012 072 357
- JP-A- 2016 050 266
- JP-A- 2016 050 266
- FORDER C ET AL: "SYNTHESIS AND AQUEOUS SOLUTION CHARACTERIZATION OF DIHYDROPHILIC BLOCK COPOLYMERS OF METHYL VINYL ETHER AND METHYL TRIETHYLENE GLYCOL VINYL ETHER", MACROMOLECULES, AMERICAN CHEMICAL SOCIETY, WASHINGTON, DC, UNITED STATES, vol. 29, no. 25, 2 December 1996 (1996-12-02), pages 8160-8169, XP000640126, ISSN: 0024-9297, DOI: 10.1021/MA9608737
- SOLARO R ET AL: "MULTIFUNCTIONAL POLYMERIC MATERIALS FOR BIOMEDICAL APPLICATIONS", MACROMOLECULAR SYMPOSIA, WILEY VCH VERLAG, WEINHEIM, DE, vol. 118, 1 June 1997 (1997-06-01), pages 603-617, XP000698064, ISSN: 1022-1360

## Description

### Technical Field

The present invention relates to the use of a copolymer as a biocompatible material and the use of a composition comprising the copolymer and a solvent as an antithrombotic coating agent, and particularly to the use of a copolymer that can provide excellent antithrombotic properties; the invention also relates to a medical device provided with the coating film formed of the copolymer.

### Background Art

It is essential for medical devices used in contact with blood, such as catheters, guide wires, stents, blood vessel prostheses, vascular bypass tubes, prosthetic valves, blood filters, plasmapheresis devices, artificial organs, oxygenator devices, dialyzers, blood transfusion apparatuses, blood circuits, and blood bags, to have antithrombotic properties to prevent coagulation of blood. Thus, it is desirable to develop an antithrombotic coating agent that provides excellent antithrombotic properties to surfaces of bases used in the medical devices.

An example of known biocompatible materials having antithrombotic properties is poly(2-methoxyethyl acrylate) (PMEA) (see PTL 1). PMEA is known to have biocompatibility, such as antithrombotic properties, due to its so-called intermediate water (water in the state where an exothermic peak due to low temperature crystal formation of water is stably observed around -40°C in the course of heating from - 100°C) which is considered as water that is weakly bound to a polymer chain by an interaction with the polymer chain.

However, since PMEA itself is a hydrophilic polymer, there are concerns about peeling or dissolution in use. Thus, in PTL 1, alkyl (meth)acrylate which is more hydrophobic than 2-methoxyethyl acrylate is copolymerized to the extent that the intermediate water is sufficiently present (9 to 17% by mole in Examples).

As a technique for improving the film formation properties of PMEA, a thin film of a polymer blend in which poly(methyl methacrylate) (PMMA) is mixed with PMEA is reported (see PTL 2). According to PTL 2, the polymer blend thin film that has a prescribed proportion of PMEA shows superior antithrombotic properties to pure PMEA.

However, in such methods of improving the hydrophilicity and film formation properties by introducing PMMA or other hydrophobic components through copolymerization, polymer blending, or other conventional techniques, the amount of the hydrophobic component cannot be increased since biocompatibility is inhibited as the amount increases, which has limited the effect of improvement.

In addition, adhesion of proteins, cells, or other biocomponents is liable to occur when a hydrophobic component is introduced, and therefore such a hydrophobic component has not been able to be used in applications where anti-fouling properties (suppression of protein adsorption and suppression of cell adhesion) are required.

On the other hand, poly(2-methoxyethyl vinyl ether) (PMOVE) is known as a biocompatible material which has an oxyethylene chain structure in a side chain and has intermediate water like PMEA, and is reported to have superior antithrombotic properties to PMEA (see PTL 3).

However, PTL 3 discloses only PMOVE as a specific example with antithrombotic properties, and does not disclose antithrombotic properties of any polymer obtained by extending the oxyethylene chain which constitutes a side chain of the vinyl ether polymer or a copolymer with a hydrophobic comonomer. Moreover, anti-fouling properties in the vinyl ether polymers were never studied.

PMOVE, which is a highly viscous oil substance and is also soluble in water at living body temperature, is difficult to be used as an antithrombotic coating agent as it is. For this reason, in PTL 3, PMOVE applied on a base is irradiated with a gamma ray to crosslink and insolubilize the PMOVE. However, such a method for forming a film is not a common method and has a problem in that the material, shape, and form of the base to be coated are limited.

As another method for forming a thin film containing PMOVE, a method in which 2-methoxyethyl vinyl ether (MOVE) is copolymerized with a vinyl ether having an alicyclic backbone such as tricyclodecane vinyl ether (TCDVE) is proposed (see PTL 4). However, in Examples of PTL 4, examples of formation of a thin film are illustrated only for a polymer having a short oxyethylene chain as a side chain, such as PMOVE and poly(2-ethoxyethyl vinyl ether)(PEOVE), and furthermore, the biocompatibility, antithrombotic properties, and anti-fouling properties of the copolymer were never evaluated.

Forder et al. (MACROMOLECULES, vol. 29, no. 25, pages 8160-8169) describe the synthesis and aqueous solution characterization of dihydrophilic block copolymers of methyl vinyl ether and methyl triethylene glycol vinyl ether.

WO 2014/163626 A2 describes compositions and methods for changing a property of a coating. The coating includes a dynamic material configured to be reversibly convertible between a hydrophobic state and a hydrophilic state, wherein transition between the hydrophilic state to the hydrophobic state occurs in an environment dependent manner. The coating also includes an environment altering material configured to alter the hydrophobic or hydrophilic state of the dynamic material.

JP 2016 050266 A relates to the problem to discover a biocompatible material having excellent film forming properties, water-insolubility, and antithrombotic properties, and provide a medical supply prepared using the same. As a solution to the problem, the document describes a biocompatible copolymer comprising at least one repeating unit (A) represented by formula (1) and at least one repeating unit (B) represented by formula (2), and a medical supply formed from the copolymer, where, R¹ is a methyl group or an ethyl group, and R² is an alicyclic hydrocarbon group.

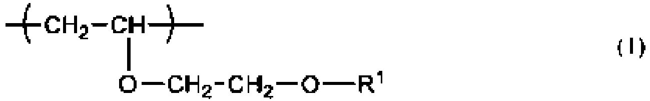

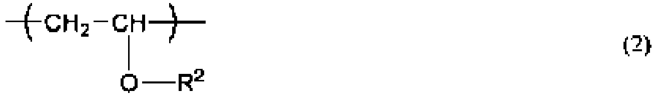

Solaro et al. (MACROMOLECULAR SYMPOSIA, vol. 118, pages 603-617) describe multifunctional polymeric materials for biomedical applications.

JP 2005 002193 A relates to the problem to provide a polymer-containing composition and an ink composition in which a functional substance such as a pigment is homogeneously dispersed. As a solution to the problem, the document describes a polymer-containing composition which includes a functional substance, a solvent and a polyvinyl ether polymer having a hydrophilic block segment. The bond between the carbon atom which is the terminal of the repeating unit of the hydrophilic block segment, on the side of not being bonded to the ether oxygen atom and constitutes the main chain of the hydrophilic block segment in the repeating unit of the hydrophilic block segment and the terminal group on the side of the carbon atom is an H-C bond or a C-C bond.

### Citation List

### Patent Literature

PTL 1: JP-A-2004-161954
PTL 2: JP-A-2013-121430
PTL 3: JP-A-2014-47347
PTL 4: Japanese Patent No. 4528601

### Summary of Invention

### Technical Problem

The present invention is made in view of the above situation, and has an object to find a biocompatible material that has excellent film formation properties and resistance to water dissolution and can be easily applied on various bases as a coating, while having excellent antithrombotic properties, and to provide an antithrombotic coating agent and an antithrombotic coating film produced by using the same, and a medical device provided with the antithrombotic coating film. The present invention also has an object to provide a biocompatible material which is also excellent in anti-fouling properties and a medical device produced by using the same.

### Solution to Problem

In general, copolymerization of a hydrophobic comonomer having a hydrophobic group leads to increase of hydrophobic interactions with biocomponents to cause adhesion of platelets and adsorption modification and activation of proteins in conditions of contact with proteins in biotissues and blood. For this reason, there has not been any example in which a polymer containing a hydrophobic vinyl ether is studied as a biocompatible material, and any copolymer with a vinyl ether having an oxyethylene chain structure, such as MOVE, has also not been studied as a biocompatible material.

However, as a result of intensive studies of copolymers of a vinyl ether containing an oxyethylene chain structure with a hydrophobic vinyl ether, the present inventors have found that a copolymer of a vinyl ether having a repetition number of oxyethylene chains of two or more with a aliphatic vinyl ether has excellent film formation properties and resistance to water dissolution, and surprisingly, also has excellent antithrombotic properties and anti-fouling properties, completing the present invention.

Specifically, the present invention concerns the use of a copolymer as a biocompatible material, wherein the copolymer is defined as follows: the copolymer contains at least one repeating unit (A) represented by the following formula (1): 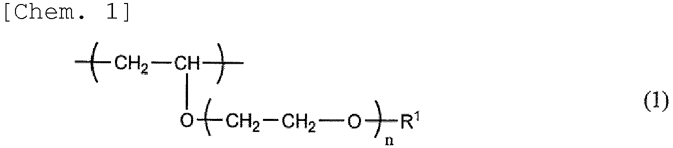 wherein,
R¹ represents a methyl group or an ethyl group, and n represents an integer of 2 to 10, and at least one repeating unit (B) represented by the following formula (2): 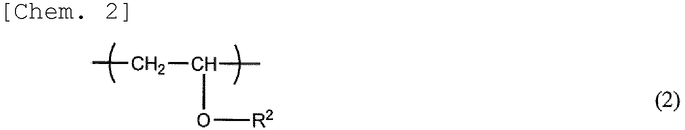 wherein,
R² represents a linear or branched alkyl group or alkenyl group having 2 to 10 carbon atoms or a monocyclic or polycyclic alkyl group or alkenyl group having 5 to 15 carbon atoms.

Present invention further concerns the use of a film formed of the copolymer defined above as an antithrombotic coating and a medical device provided with an antithrombotic coating film formed of the copolymer defined above

### Advantageous Effects of Invention

The copolymer used in the present invention can be easily applied on various bases as a coating, has excellent film formation properties and resistance to water dissolution, and can form an antithrombotic coating film. The copolymer can be suitably used as a biocompatible material.

Accordingly, the copolymer is useful as a component of an antithrombotic coating agent, and an antithrombotic coating film formed of the copolymer has excellent antithrombotic properties and can significantly suppress coagulation of blood even when it is in contact with blood over a long period of time. Thus, the copolymer is highly useful for a coating film of a medical device.

A coating film formed of the copolymer used in the present invention can prevent adhesion of biocomponents, such as cells, and can be suitably used in various medical devices as a material having anti-fouling properties.

### Description of Embodiments

### <Copolymer>

The copolymer used in the present invention contains at least one repeating unit (A) represented by the formula (1) and at least one repeating unit (B) represented by the formula (2) and thereby exhibits excellent biocompatibility, such as antithrombotic properties and anti-fouling properties.

Examples of monomers providing the repeating unit (A) of the formula (1) include hydrophilic vinyl ethers represented by the following formula (3): 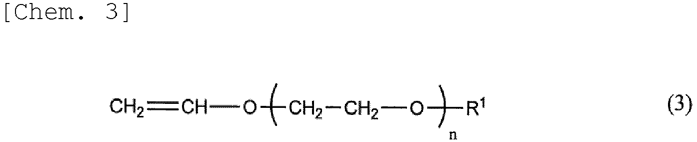 wherein R¹ is a methyl group or ethyl group, the repetition number n of the oxyethylene chains is an integer of 2 to 10, preferably an integer of 2 to 6, more preferably an integer of 2 to 4, and particularly preferably 2 or 3.

Specific examples of hydrophilic vinyl ethers represented by the formula (3) include 2-(2-methoxyethoxy)ethyl vinyl ether (synonym: diethylene glycol monomethyl monovinyl ether), 2-(2-ethoxyethoxy)ethyl vinyl ether (synonym: diethylene glycol monoethyl monovinyl ether; hereinafter referred to as "EOEOVE"), 2-[2-(2-methoxyethoxy)ethoxy]ethyl vinyl ether (synonym: triethylene glycol monomethyl monovinyl ether; hereinunder referred to as "TEGVE"), and 2-[2-(2-ethoxyethoxy)ethoxy]ethyl vinyl ether (synonym: triethylene glycol monoethyl monovinyl ether). Among them, EOEOVE and TEGVE are preferred in terms of film formation properties and resistance to water dissolution, and EOEOVE is particularly preferred due to its superior antithrombotic properties.

Examples of monomers providing the repeating unit (B) include hydrophobic vinyl ethers represented by the following formula (4)

[Chem. 4] CH₂=CH-OR² (4)

wherein R² is as defined above.

The linear or branched alkyl group or alkenyl group preferably has 2 to 8 carbon atoms, and more preferably has 2 to 6 carbon atoms.

Specific examples of linear or branched alkyl groups or alkenyl groups include linear or branched alkyl groups, such as an ethyl group, an n-propyl group, an isopropyl group, an n-butyl group, an isobutyl group, a tert-butyl group, a 1-pentyl group, a 2-pentyl group, a 3-pentyl group, a 1-(2-methyl)-butyl group, a 2-(2-methyl)-butyl group, a 1-(3-methyl)-butyl group, a 2-(3-methyl)-butyl group, a (2,2-dimethyl)-propyl group, a 1-hexyl group, a 2-hexyl group, a 3-hexyl group, a 1-heptyl group, a 2-heptyl group, a 3-heptyl group, a 4-heptyl group, a 1-octyl group, and a 1-(2-ethyl)-hexyl group; and linear or branched alkenyl groups, such as a vinyl group, a 1-propenyl group, an allyl group, a 2-butenyl group, a 3-butenyl group, an isopropenyl group, an isobutenyl group, a 1-pentenyl group, a 2-pentenyl group, a 3-pentenyl group, a 4-pentenyl group, a 1-hexenyl group, a 2-hexenyl group, a 3-hexenyl group, a 4-hexenyl group, and a 5-hexenyl group.

The monocyclic or polycyclic alkyl group or alkenyl group has 5 to 15 carbon atoms.

Specific examples of monocyclic or polycyclic alkyl groups or alkenyl groups include monocyclic alkyl groups or alkenyl groups, such as a cyclopentyl group, a cyclopentylmethyl group, a methylcyclopentyl group, a dimethylcyclopentyl group, a cyclohexyl group, a cyclohexylmethyl group, a methylcyclohexyl group, a dimethylcyclohexyl group, a cyclohexenyl group, a cycloheptyl group, a cyclooctyl group, a cyclononyl group, a cyclodecyl group, a cycloundecyl group, a cyclododecyl group, a cyclotridecyl group, a cyclotetradecyl group, and a cyclopentadecyl group; and polycyclic alkyl groups or alkenyl, such as a bicyclohexyl group, a decahydronaphthyl group, a norbornyl group, a methylnorbornyl group, an isobornyl group, an adamantyl group, a tricyclodecanyl group, a tricyclodecenyl group, and a tetracyclododecyl group.

Among the aliphatic hydrocarbon groups, an n-butyl group and a tricyclodecanyl group are preferred in terms of film formation properties, resistance to water dissolution, and antithrombotic properties, and an n-butyl group is particularly preferred due to its superior antithrombotic properties.

The copolymer used in the present invention can be prepared by polymerizing the hydrophilic vinyl ether (3) and the hydrophobic vinyl ether (4) according to an ordinary method. As a polymerization method, living cation polymerization is particularly preferred for obtaining a copolymer having a desired composition ratio and molecular weight in a highly reproducible manner. Since the molecular weight of a copolymer is determined substantially uniquely by the molar ratio of a monomer and a polymerization initiator in a living cation polymerization method, the molecular weight of the copolymer can be controlled arbitrarily over a wide range by varying the amount of monomers used and the amount of a polymerization initiator used.

Any polymerization initiator that can promote cation polymerization in a living manner can be used in living cation polymerization without any limitation. For example, HI/I₂-type initiators (for example, JP-A-60-228509), and polymerization initiators obtained by combining a Lewis acid catalyst (an organic aluminum compound or the like) and an additive, such as a base (ether, ester, or the like) (for example, Japanese Patent No. 3096494, JP-B-7-2805, JP-A-62-257910, JP-A-1-108202, and JP-A-1-108203) are suitably used as a living cation polymerization initiator for vinyl ethers.

The amount of a polymerization initiator used is preferably 0.001 to 20% by mole based on the total amount of the raw monomers, more preferably 0.01 to 10% by mole, and particularly preferably 1% by mole or less.

The living cation polymerization reaction is preferably performed in the presence of an appropriate organic solvent, but may be performed in the absence of an organic solvent. Examples of usable organic solvents include aromatic hydrocarbon solvents, such as benzene, toluene, and xylene; aliphatic hydrocarbon solvents, such as propane, n-butane, isobutane, n-pentane, isopentane, n-hexane, n-heptane, n-octane, isooctane, decane, hexadecane, and cyclohexane; halogenated hydrocarbon solvents, such as methylene chloride, ethylene chloride, and carbon tetrachloride; ether solvents, such as diethyl ether, dibutyl ether, tetrahydrofuran (THF), dioxane, and ethylene glycol diethyl ether. The organic solvents may be used alone or in combination of two or more thereof as needed. Among the organic solvents, hydrocarbon solvents, such as aromatic hydrocarbon solvents and aliphatic hydrocarbon solvents, are preferred, and toluene or cyclohexane is particularly preferred.

The polymerization temperature in the polymerization reaction varies depending on the types of the polymerization initiator, monomer, solvent used and the like, but is generally -80 to 150°C, preferably -50 to 100°C, and particularly preferably -20 to 80°C. The polymerization time varies depending on the polymerization initiator, monomer, solvent, reaction temperature used, but is generally approximately 10 minutes to 100 hours. The polymerization reaction can be suitably performed by any of a batch method and a continuous method. After the polymerization reaction, a purification treatment may be performed according to a known method to remove unreacted monomers as needed.

The composition ratio (molar ratio) of the repeating unit (A) to the repeating unit (B) in the copolymer used in the present invention can be arbitrarily selected in the range where the film formation properties and antithrombotic properties are not impaired, but surprisingly in the copolymer used in the present invention, a copolymer having a lower proportion of the repeating unit (A) which is a hydrophilic unit exhibits higher antithrombotic properties. Accordingly, the composition ratio (molar ratio) of the repeating unit (A) to the repeating unit (B) is preferably in the range of 90/10 to 1/99, more preferably in the range of 70/30 to 3/97, and particularly preferably in the range of 50/50 to 5/95.

The molecular weight of the copolymer used in the present invention is approximately 1,000 to 1,000,000, preferably 2,000 to 500,000, and more preferably 3,000 to 300,000 as a weight average molecular weight (Mw) determined, for example, from a standard polystyrene calibration curve by gel permeation chromatography (GPC).

The ratio (Mw/Mn) of the weight average molecular weight (Mw) to the number average molecular weight (Mn) of the copolymer used in the present invention is preferably 1.0 to 5.0, more preferably 1.0 to 3.0, and particularly preferably 1.0 to 1.5. Such Mw and Mw/Mn in the above ranges provide a copolymer having excellent coating performance and excellent antithrombotic properties.

The copolymer used in the present invention is obtained by copolymerizing the hydrophilic vinyl ether and the hydrophobic vinyl ether as described above. There is no limitation in the sequencing manner of the monomer units, and the copolymer may be any of a random copolymer and a block copolymer. Examples of block copolymers include block polymers, such as a di-block type (A-B), a tri-block type (A-B-A or B-A-B), and a multi-branched star type ([B-A]ₙ, [A-B]ₙ, or AₙBₘ; wherein n and m are numbers of branches). From the viewpoint of uniformly dispersing the hydrophilic units, random copolymers are preferred.

### <Antithrombotic Coating Agent>

Since the copolymer used in the present invention obtained as described above has excellent biocompatibility, such as antithrombotic properties and anti-fouling properties, the copolymer can be suitably used as a biocompatible material and, for example, can be found into an antithrombotic coating agent containing the copolymer as an active constituent. The antithrombotic coating agent can be prepared by blending an appropriate solvent with the copolymer.

The type and concentration of the solvent in the antithrombotic coating agent can be appropriately selected according to the composition and molecular weight of the copolymer and the type and surface properties of the base to be coated.

As a solvent in the antithrombotic coating agent, for example, organic solvents mentioned as a polymerization solvent in living cation polymerization can be used. In addition, alcohol solvents, such as methanol, ethanol, and isopropanol; ketone solvents, such as acetone, methyl ethyl ketone, and methyl amyl ketone; and ester solvents, such as methyl acetate, ethyl acetate, amyl acetate, and ethyl lactate can also be suitably used. Among the organic solvents, hydrocarbon solvents, such aromatic hydrocarbon solvents and aliphatic hydrocarbon solvents, ether solvents, and mixed solvents thereof are preferred, and toluene, cyclohexane, THF, and mixed solvents thereof are particularly preferred. The organic solvents may be used alone or in combination of two or more thereof as needed.

The concentration of the solvent in the antithrombotic coating agent is preferably 99.95 to 90 parts by mass of the solvent relative to 0.05 to 10 parts by mass of the copolymer.

The antithrombotic coating agent described above is used for forming an antithrombotic coating film containing the copolymer on various bases, in particular, on bases that may be brought into contact with blood.

The method for forming an antithrombotic coating film using the antithrombotic coating agent is appropriately selected from, but not limited to, known methods, such as application methods, spraying methods, dipping methods, spin coating methods according to the material, shape, and form of the base. The antithrombotic coating film can be formed by a simple operation in which, for example, a base is immersed in an antithrombotic coating agent that contains the copolymer and an organic solvent, and then the coating is dried by air or dried with heating.

Since the antithrombotic coating agent has a simple configuration as described above, there is no limitation in the material, shape, and form of the base on which the antithrombotic coating film is formed, and the antithrombotic coating agent can be used on a base of any shape or form, such as a film, sheet, plate, fiber, nonwoven fabric, porous body, tube, hollow yarn or fiber, particle, and powder.

Examples of materials of the base include synthetic resins, such as polyolefin, for example, polypropylene and polyethylene, nylon, polyester, polyacrylonitrile, halogenated polyolefin, polystyrene, polycarbonate, polyvinyl chloride, polyurethane, polyamide, polysulfone, polyethersulfone, poly(meth)acrylate, an ethylene-vinyl alcohol copolymer, and a butadiene-acrylonitrile copolymer, and blend polymers thereof; natural polymers, such as cotton and hemp; inorganic materials, such as metals, ceramics, and glasses; and composite materials thereof, and these materials can be used.

Preferred examples of objects on which an antithrombotic coating film is formed from the antithrombotic coating agent include medical devices. Many medical implements are brought into contact with blood, and in such a case, adhesion of platelets and coagulation of blood by aggregation have to be prevented, and therefore such medical implements are required to be provided with an antithrombotic coating film. Accordingly, in the medical devices, a part, and preferably, the whole of a section to be in contact with blood is preferably coated with the antithrombotic coating film.

The medical device of the present invention which is treated with an antithrombotic coating agent as described above is provided with the antithrombotic coating film, and thus can be suitably used particularly for applications in which a medical device is used in direct contact with blood. Specifically, the medical devices can be used for various applications, such as intracorporeal implant type artificial organa or therapeutic implements, extracorporeal circulation type artificial organs, catheters (for example, cardiovascular catheters, such as angiography catheters, guide wires, and PTCA catheters, digestive organ catheters and tubes, such as gastric tube catheters, gastrointestinal catheters, and esophageal tubes, and urological catheters, such as urethral catheters and ureteral catheter), blood vessel prostheses, vascular bypass tubes, prosthetic valves, blood filters, plasmapheresis devices, blood transfusion apparatuses, extracorporeal circuits for blood, blood bag, hemostatics, and biotissue adhesion materials. An antithrombotic coating film, which is formed on a part or the whole of a section of a medical device to be in contact with blood, can be used as a method for preventing formation of thrombi.

### Examples

The present invention will be described more specifically with reference to Examples and Synthetic Examples, but the present invention is never limited to the examples. In the examples, the composition ratios of copolymers were determined from ¹H-NMR analysis results, and the weight average molecular weights (Mw) and molecular weight distributions (Mw/Mn) thereof were determined from molecular weight analysis results (in terms of polystyrene) in GPC. The analysis apparatuses and measurement conditions are as follows.

### (NMR)

• Apparatus: AVANCE400 manufactured by Burker
• Solvent: deuterated acetone
• Measurement temperature: 30°C

### (GPC)

• Apparatus: "HLC-8320GPC" manufactured by TOSOH Corporation
• Detector: RI detector
• Mobile phase: tetrahydrofuran
• Flow rate: 1 mL/min
• Column: 3 × "Shodex LF-804" manufactured by SHOWA DENKO K.K.
· Column temperature: 40°C

### Synthetic Example 1

Synthesis of N-butyl vinyl ether/diethylene glycol monoethyl monovinyl ether random copolymer (NBVE-ran-EOEOVE):
Into a 300-mL three neck flask with a three-way stopcock previously subjected to dewatering with heat at 300°C under dry nitrogen atmosphere for 10 minutes, 181 mL of toluene as a solvent, 76.4 mL of ethyl acetate as an added base, 4.0 mL of diethylene glycol monoethyl monovinyl ether (EOEOVE) as a hydrophilic vinyl ether, 28.2 mL of N-butyl vinyl ether (NBVE) as a hydrophobic vinyl ether, 4 mM (0.45 mL) of an acetic acid adduct of isobutyl vinyl ether as an initiator species were added, and the mixture was stirred well.

Next, the flask was kept at 0°C, and 8 mM (8.8 mL) of Et_{1.5}AlCl_{1.5} was added as a Lewis acid catalyst to start polymerization, and a reaction was carried out for 90 minutes.

The polymerization was stopped by methanol containing a small amount of sodium methoxide (1 M). To the solution in which the reaction stopped, 5% by mass of an ion exchange resin [trade name: Amberlyst MSPS2-1·DRY, manufactured by ORGANO CORPORATION] was added and the mixture was stirred at room temperature for 1 hour. Next, the solution was passed through Celite and a filter with a pore size of 1 um, and was concentrated under reduced pressure by an evaporator to obtain a target random copolymer (copolymer C). The composition ratio, weight average molecular weight (Mw), and molecular weight distribution (Mw/Mn) of the resulting copolymer C are shown in Table 1.

A 1% by mass aqueous solution of the resulting copolymer C was prepared, and the solubility into water at 25°C was visually checked. As a result, the aqueous solution was divided into an aqueous phase and a polymer phase, which confirmed insolubilization in water due to introduction of a hydrophobic unit.

### Synthetic Examples 2 to 14

Copolymers A, B, D to N shown in Table 1 were produced by performing synthesis based on Synthetic Example 1 while using EOEOVE or TEGVE as a hydrophilic vinyl ether and using NBVE or tricyclodecanyl vinyl ether (TCDVE) as a hydrophobic vinyl ether and varying the amount of the initiator species and the composition ratio. The resulting copolymers were evaluated for the solubility in water by the same operation as in Synthetic Example 1. The composition ratio, molecular weight (Mw), molecular weight distribution (Mw/Mn), and solubility in water of each copolymer are shown in Table 1.

### Synthetic Examples 15 to 17

Homopolymers O, P, and Q shown in Table 1 were produced by polymerizing each of EOEOVE, NBVE, and TCDVE alone based on Synthetic Example 1. The resulting homopolymers were evaluated for the solubility in water by the same operation as in Synthetic Example 1. The evaluation results of the molecular weight (Mw), molecular weight distribution (Mw/Mn), and solubility in water of each homopolymer are shown in Table 1.

**[Table 1]**

| Sample name | Chemical name | Hydrophilic unit (mol%) | Mw | Mw/Mn | Solubility in water (25°C) |
|---|---|---|---|---|---|
| A | NBVE-ran-EOEOVE | 10 | 3400 | 1.43 | Insoluble |
| B | | | 4200 | 1.48 | Insoluble |
| C | | | 11500 | 1.12 | Insoluble |
| D | | | 14700 | 1.11 | Insoluble |
| E | | | 24300 | 1.16 | Insoluble |
| F | | 20 | 3600 | 1.20 | Insoluble |
| G | | | 11700 | 1.14 | Insoluble |
| H | | | 14100 | 1.10 | Insoluble |
| I | | | 23300 | 1.08 | Insoluble |
| J | NBVE-ran-TEGVE | 10 | 9900 | 1.10 | Insoluble |
| K | NBVE-block-TEGVE | 10 | 14600 | 1.12 | Insoluble |
| L | TCDVE-ran-TEGVE | 30 | 35200 | 1.15 | Insoluble |
| M | TCDVE-block-TEGVE | 30 | 44100 | 1.09 | Insoluble |
| N | TEGVE-block-NBVE- block-TEGVE | 30 | 11700 | 1.14 | Insoluble |
| O | EOEOVE | 100 | 11600 | 1.21 | Soluble |
| P | NBVE | 0 | 25200 | 1.14 | Insoluble |
| Q | TCDVE | 0 | 29600 | 1.08 | Insoluble |

### (Solubility in Water)

· Insoluble: divided into aqueous phase and polymer phase.
· Soluble: uniformly dissolved.

### Examples 1 to 14 and Comparative Examples 1 to 3

### Blood Compatibility Test:

In order to test blood compatibility, polyethylene terephthalate (PET) plates (Examples 1 to 14) surfaces of which were coated with the respective copolymers A to N according to the Synthetic Examples, and PET plates (Comparative Examples 1 to 3) surfaces of which were coated with the respective homopolymers O to Q as comparative examples were subjected to a platelet adhesion test.

The surface coating of the PET plates with polymers according to Examples and Comparative Examples was performed by applying a 0.2 wt/vol% toluene solution of each polymer obtained in the Synthetic Examples on a surface of the PET plate and evaporating the solvent for exsiccation.

0.2 mL of a human fresh platelet-rich plasma treated for anti-coagulation with sodium citrate was added dropwise with a pippete to the PET surface coated with each polymer and the PET plate coated with no polymer (blank), and the plates were allowed to stand at 37°C for 60 minutes. Subsequently, the plates were rinsed with a phosphate buffer solution and were fixed with glutaraldehyde. Then, the sample surfaces were each observed by a scanning electron microscope to count the number of platelets that adhered in an area of 1 × 104 µm². Although peeling of a coating layer was observed in the case of the polymer O (homopolymer of EOEOVE), the evaluation was continued as it was.

The results of the blood compatibility test are shown in Table 2. It was shown that the PET plate a surface of which was coated with each of the copolymers A to N provides a smaller number of platelet adhesion as compared with Comparative Examples and the blank PET plate.

**[Table 2]**

| Example | Sample name | Chemical name | Hydrophilic unit (mol%) | Platelet adhesion (×10⁵ cells/cm2) |
|---|---|---|---|---|
| Example 1 | A | NBVE-ran-EOEOVE | 10 | 0.32 |
| Example 2 | B | | | 0.06 |
| Example 3 | C | | | 0.05 |
| Example 4 | D | | | 0.30 |
| Example 5 | E | | | 0.06 |
| Example 6 | F | | 20 | 0.05 |
| Example 7 | G | | | 0.20 |
| Example 8 | H | | | 0.50 |
| Example 9 | I | | | 2.00 |
| Example 10 | J | NBVE-ran-TEGVE | 10 | 0.20 |
| Example 11 | K | NBVE-block-TEGVE | 10 | 0.83 |
| Example 12 | L | TCDVE-ran-TEGVE | 30 | 0.20 |
| Example 13 | M | TCDVE-block-TEGVE | 30 | 1.03 |
| Example 14 | N | TEGVE-block-NBVE- block-TEGVE | 30 | 0.50 |
| Comparative Example 1 | Q | EOEOVE | 100 | 6.70 |
| Comparative Example 2 | P | NBVE | 0 | 2.80 |
| Comparative Example 3 | Q | TCDVE | 0 | 6.80 |
| Blank | PET base | - | - | 9.75 |

### Examples 15 to 18

### Cancer Cell Adhesion Test:

PET plates coated with the copolymers A, B, and C were fabricated in the same manner as in Examples 1 to 3 and 1.0 mL of a cancer cell suspension (prepared at 10,000 cells/mL in a medium having serum added at 10%) was added with a pippete to PET surfaces coated with the copolymers and a PET plate coated with no copolymer (blank), and the plates were allowed to stand at 37°C for 60 minutes. As the cancer cells, a human fibrosarcoma cell line, HT-1080 was used. Subsequently, the plate was rinsed with a physiological buffer saline solution and the number of cells adhered on the sample surface was counted. For facilitating the counting, the cells were fixed with formaldehyde, then the cell nuclei were stained with 4',6-diamino-2-phenylindole (DAPI), and the number of the cell nuclei was counted using confocal laser scanning microscope (Olympus FV-1000) and the count was taken as the cell number.

The results of the cell adhesion test are shown in Table 3. Although the copolymers A, B, and C contain hydrophobic units at 90% by mole, the cell adhesion numbers thereof were found to be very small.

**[Table 3]**

| Sample name | Chemical name | Hydrophilic unit (mol%) | Mw | Mw/Mn | HT1080 adhesion (×10⁴ cells/cm2) |
|---|---|---|---|---|---|
| A | NBVE-ran-EOEOVE | 10 | 3400 | 1.43 | 0.03 |
| B | | | 4200 | 1.48 | 0.19 |
| C | | | 11500 | 1.12 | 0.03 |
| PET base | - | - | - | - | 1.30 |

### Industrial Applicability

As described above, coating films formed of the copolymer used in the present invention are excellent in film formation properties and resistance to water dissolution, and can prevent adhesion of platelets, and can prevent production of thrombi caused in turn by the adhesion. Thus, the copolymer used in the present invention is useful as a biocompatible material conforming to ISO10993.

In addition, an antithrombotic coating film formed by using the above antithrombotic coating agent containing the copolymer used in the present invention has excellent antithrombotic properties, and particularly when the antithrombotic coating film is formed on a medical device that is brought into contact with blood, production of thrombi can be prevented.

Furthermore, a coating film formed of the copolymer used in the present invention can prevent adhesion of biocomponents, such as cells, and can be suitably used as a material having anti-fouling properties in various medical devices.

Accordingly, the present invention is highly useful in the medical field and the field of production of medical devices.

## Claims

1. Use of a copolymer as a biocompatible material, wherein the copolymer is defined as follows:
the copolymer comprises
at least one repeating unit (A) represented by formula (1) : 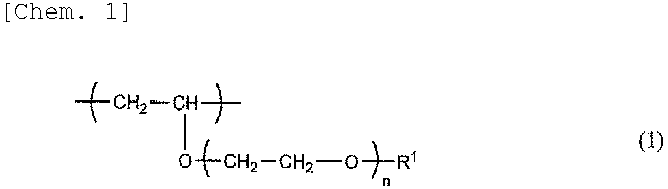
wherein R¹ represents a methyl group or an ethyl group, and n represents an integer of 2 to 10, and
at least one repeating unit (B) represented by formula (2) : 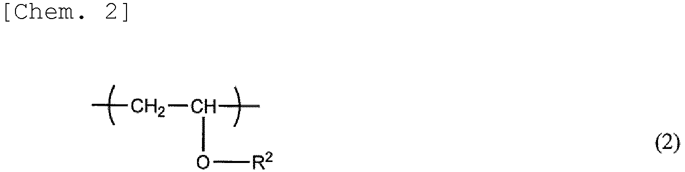
wherein R² represents a linear or branched alkyl group or alkenyl group having 2 to 10 carbon atoms or a monocyclic or polycyclic alkyl group or alkenyl group having 5 to 15 carbon atoms.

2. The use according to claim 1, wherein, in the copolymer, the composition ratio (molar ratio) of the repeating unit (A) to the repeating unit (B) is 90/10 to 1/99.

3. The use according to claim 1 or 2, wherein, in the copolymer, the group R² in the repeating unit (B) is a linear or branched alkyl group or alkenyl group having 2 to 10 carbon atoms.

4. Use of a composition comprising the copolymer defined in any one of claims 1 to 3 and an organic solvent as an antithrombotic coating agent.

5. The use according to claim 4, wherein the organic solvent is one or two or more selected from the group consisting of an aromatic hydrocarbon, an aliphatic hydrocarbon, a halogenated hydrocarbon, an ether, an alcohol, a ketone, and an ester.

6. The use according to claim 4 or 5, wherein the amount of the copolymer is 0.05 to 10 parts by mass and the amount of the organic solvent is 99.95 to 90 parts by mass.

7. Use of a film formed of the copolymer defined in any one of claims 1 to 3 as an antithrombotic coating.

8. A medical device provided with an antithrombotic coating film formed of the copolymer defined in any one of claims 1 to 3.

9. The medical device according to claim 8, wherein a part or the whole of a section to be in contact with blood is coated with the antithrombotic coating film.

10. The medical device according to claim 8 or 9, which is an intracorporeal implant type artificial organum or therapeutic implement, an extracorporeal circulation type artificial organ, a catheter, a blood vessel prosthesis, a vascular bypass tube, a prosthetic valve, a blood filter, a plasmapheresis device, a blood transfusion apparatus, or an extracorporeal circuit for blood.

## Patentansprüche

1. Verwendung eines Copolymers als biokompatibles Material, wobei das Copolymer wie folgt definiert ist:
das Copolymer umfasst
mindestens eine sich wiederholende Einheit (A), dargestellt durch die Formel (1): 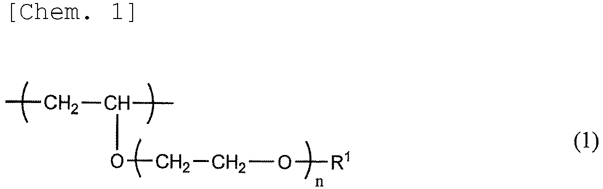
worin R¹ eine Methylgruppe oder eine Ethylgruppe darstellt und n eine ganze Zahl von 2 bis 10 ist, und
mindestens eine sich wiederholende Einheit (B), dargestellt durch die Formel (2): 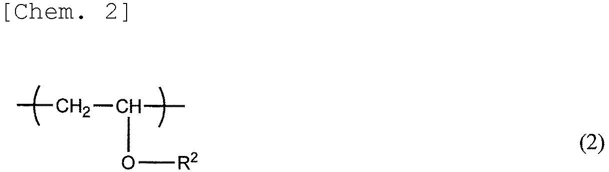
worin R² eine lineare oder verzweigte Alkylgruppe oder Alkenylgruppe mit 2 bis 10 Kohlenstoffatomen oder eine monocyclische oder polycyclische Alkylgruppe oder Alkenylgruppe mit 5 bis 15 Kohlenstoffatomen darstellt.

2. Verwendung nach Anspruch 1, wobei in dem Copolymer das Zusammensetzungsverhältnis (Molverhältnis) der sich wiederholenden Einheit (A) zu der sich wiederholenden Einheit (B) 90/10 bis 1/99 beträgt.

3. Verwendung nach Anspruch 1 oder 2, wobei in dem Copolymer die Gruppe R² in der sich wiederholenden Einheit (B) eine lineare oder verzweigte Alkylgruppe oder Alkenylgruppe mit 2 bis 10 Kohlenstoffatomen ist.

4. Verwendung einer Zusammensetzung, die das in einem der Ansprüche 1 bis 3 definierte Copolymer und ein organisches Lösungsmittel enthält, als antithrombotisches Beschichtungsmittel.

5. Verwendung nach Anspruch 4, wobei das organische Lösungsmittel eines oder zwei oder mehr ist, ausgewählt aus der Gruppe bestehend aus einem aromatischen Kohlenwasserstoff, einem aliphatischen Kohlenwasserstoff, einem halogenierten Kohlenwasserstoff, einem Ether, einem Alkohol, einem Keton und einem Ester.

6. Verwendung nach Anspruch 4 oder 5, wobei die Menge des Copolymers 0,05 bis 10 Massenteile und die Menge des organischen Lösungsmittels 99,95 bis 90 Massenteile beträgt.

7. Verwendung eines Films, der aus dem in einem der Ansprüche 1 bis 3 definierten Copolymer gebildet ist, als antithrombotische Beschichtung.

8. Medizinische Vorrichtung, die mit einem antithrombotischen Beschichtungsfilm versehen ist, der aus dem in einem der Ansprüche 1 bis 3 definierten Copolymer gebildet ist.

9. Medizinische Vorrichtung nach Anspruch 8, wobei ein Teil oder der gesamte Abschnitt, der mit Blut in Kontakt kommt, mit dem antithrombotischen Beschichtungsfilm beschichtet ist.

10. Medizinische Vorrichtung nach Anspruch 8 oder 9, bei der es sich um ein künstliches Organ oder therapeutisches Gerät vom Typ eines intrakorporalen Implantats, ein künstliches Organ vom Typ eines extrakorporalen Kreislaufs, einen Katheter, eine Blutgefäßprothese, einen vaskulären Bypass-Schlauch, eine Ventilprothese, einen Blutfilter, eine Plasmapheresevorrichtung, ein Bluttransfusionsgerät oder einen extrakorporalen Blutkreislauf handelt.

## Revendications

1. Utilisation d'un copolymère comme matériau biocompatible, le copolymère étant défini comme suit :
le copolymère comprend
au moins une unité répétitive (A) représentée par la formule (1) : 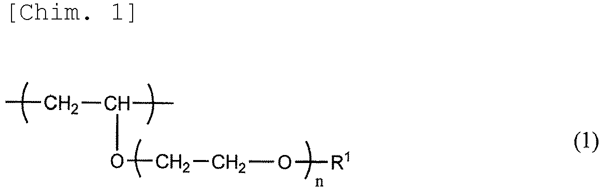
dans laquelle R¹ représente un groupe méthyle ou un groupe éthyle, et n représente un nombre entier de 2 à 10, et
au moins une unité répétitive (B) représentée par la formule (2) : 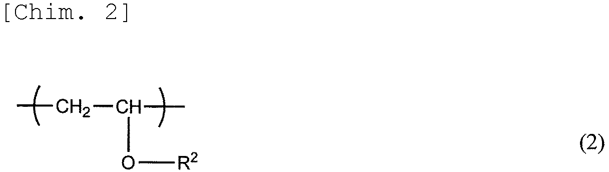
dans laquelle R² représente un groupe alkyle ou alcényle linéaire ou ramifié ayant de 2 à 10 atomes de carbone ou un groupe alkyle ou alcényle monocyclique ou polycyclique ayant de 5 à 15 atomes de carbone.

2. Utilisation selon la revendication 1, dans laquelle, dans le copolymère, le rapport de composition (rapport molaire) de l'unité répétitive (A) à l'unité répétitive (B) est de 90/10 à 1/99.

3. Utilisation selon la revendication 1 ou 2, dans laquelle, dans le copolymère, le groupe R² dans l'unité de répétition (B) est un groupe alkyle linéaire ou ramifié ou un groupe alcényle ayant de 2 à 10 atomes de carbone.

4. Utilisation d'une composition comprenant le copolymère défini dans l'une quelconque des revendications 1 à 3 et un solvant organique comme agent d'enrobage antithrombotique.

5. Utilisation selon la revendication 4, dans laquelle le solvant organique est un ou deux ou plus choisis dans le groupe constitué d'un hydrocarbure aromatique, d'un hydrocarbure aliphatique, d'un hydrocarbure halogéné, d'un éther, d'un alcool, d'une cétone et d'un ester.

6. Utilisation selon la revendication 4 ou 5, dans laquelle la quantité de copolymère est de 0,05 à 10 parties en masse et la quantité de solvant organique est de 99,95 à 90 parties en masse.

7. Utilisation d'un film formé du copolymère défini dans l'une quelconque des revendications 1 à 3 comme revêtement antithrombotique.

8. Dispositif médical muni d'un film d'enrobage antithrombotique formé du copolymère défini dans l'une quelconque des revendications 1 à 3.

9. Dispositif médical selon la revendication 8, dans lequel une partie ou la totalité d'une section destinée à être en contact avec le sang est recouverte du film d'enrobage antithrombotique.

10. Dispositif médical selon la revendication 8 ou 9, qui est un organe artificiel ou un instrument thérapeutique de type implant intracorporel, un organe artificiel de type circulation extracorporelle, un cathéter, une prothèse de vaisseau sanguin, un tube de dérivation vasculaire, une valve prothétique, un filtre à sang, un dispositif de plasmaphérèse, un appareil de transfusion sanguine, ou un circuit extracorporel pour le sang.
